# EUROPÄISCHE PATENTSCHRIFT

(11) **EP 0 625 581 B1**
(45) Veröffentlichungstag und Bekanntmachung des Hinweises auf die Patenterteilung: **30.12.1998**
(21) Anmeldenummer: 94107040.1
(22) Anmeldetag: 05.05.1994
(51) Int. Cl.: C12Q 1/04, C12Q 1/18

(54) **Verfahren zum optischen Nachweis von Mikroorganismen, ihrer Identifizierung und der Empfindlichkeitstestung gegen Antibiotika unter Verwendung eines Redoxindikatorsystems**
Method for the optical detection of microorganisms, their identification and antibiotic sensitivity testing using a redox-indicator system
Procédé pour la détection optique des micro-organismes, leur identification et test de sensibilité contre les antibiotiques utilisant une système à indicateur redox

(30) Priorität: 17.05.1993 DE 4316394
(43) Veröffentlichungstag der Anmeldung: 23.11.1994
(73) Patentinhaber: BIOTEST AG, D-63303 Dreieich (DE)
(72) Erfinder: Horn, Jürgen, Dr., D-63329 Egelsbach (DE)
(74) Vertreter: Beil, Hans Chr., Dr.

(56) Entgegenhaltungen:
- EP-A- 0 505 023
- WO-A-90/08196

## Beschreibung

Die Erfindung betrifft ein Verfahren zum optischen Nachweis des Wachstums von Mikroorganismen wie Pilzen, Hefen und Bakterien, insbesondere Mykobakterien, sowie zur Identifizierung und Empfindlichkeitstestung gegen Antibiotika durch visuelle Ermittlung des Farbumschlags eines spezifischen Redoxindikatorsystems, umfassend Methylenblau und Resazurin bzw. FeIII- oder FeII-Salze in Kombination mit K₃Fe(CN)₆ bzw. K₄Fe(CN)₆ oder Natriumwolframat (Na₂WO₄).

Mikroorganismen aus verschiedenem Probematerial (Urin, Wunden, Abszesse, Blut, Sputum) werden als Primärkultur isoliert. Das Wachstum ist in Form von Bakterienkolonien auf den zur primären Isolierung verwendeten Agarplatten sichtbar.

Zur Identifizierung werden Agarplatten oder Bouillons mit verschiedenen Substraten verwendet, in denen das Wachstum der Bakterien anhand der Trübung oder von Farbumschlägen beurteilt wird. Je nach verwendetem Substrat ergeben sich dabei zahlreiche verschiedene Farbumschläge, die sogenannte bunte Reihe (DIN 58 959, Teil 9, Beiblatt 1).

Bekannt ist weiter ein einheitlicher Farbumschlag bei der Verwendung von 95 verschiedenen Substraten mit dem Redoxindikator Tetrazoliumviolett als Nachweis der metabolischen Aktivität der Bakterien (Bochner, American Clinical Laboratory Seite 14, April 1991).

Zur Empfindlichkeitsprüfung wird der Agardiffusionstest oder eine Bouillon Dilutionsmethode wie die Mikrodilution verwendet (DIN 58 940, Teil 1, 2, 8). Gemessen wird hierbei die Trübung (Dilutionsverfahren) bzw. das Wachsen von Kolonien (Agardiffusion). Weiter ist die Verwendung des Redoxindikators Resazurin zum visuellen Nachweis des bakteriellen Wachstums bei der Sensibilitätstestung mittels Farbumschlags (WO90/08196) bekannt. Da die Farbe des Redoxindikators pH-abhängig ist, wird vorzugsweise ein alkalischer Puffer gewählt. Um darüberhinaus einen Farbwechsel durch das Nährmedium selbst (ohne bakterielles Wachstum) zu verhindern, ist ein Redoxstabilisator (Kaliumhexacyanoferrat) erforderlich.

Die EP-B 0135023 offenbart den Nachweis von intrazellulären Oxidoreduktasen mit Redoxindikatoren zur Sensibilitätstestung von ausschließlich bakteriziden Antibiotika mit Primärwirkung auf die Mureinbiosynthese. Hier werden nur durch Zelltod freigesetzte Enzyme nachgewiesen, nicht jedoch das Wachstum (bzw. der Metabolismus der wachsenden Zelle) an sich.

In J. of Dairy Sciences S. 705, 1935, wird beschrieben, daß Resazurin als Redoxindikator zum Nachweis der bakteriellen Kontamination in Milch wesentlich schneller umschlägt als Methylenblau (siehe auch Sigma-Aldrich Handbook of Stains and Dyes, S. 622). Dies hängt offensichtlich mit dem Farbumschlag von blau nach rot hinsichtlich Resazurin zusammen, während Methylenblau nur einen Übergang von blau nach farblos aufzeigt.

Wie erwähnt, ist die in der EP-B 0135023 beschriebene Methode nur bei Antibiotika, wie z.B. Penicillin, möglich, welche bakterizid auf die Mureinbiosynthese wirken. Sie versagt jedoch bei anderen Bakteriostatika, wie z.B. Tetracyclin, und ist daher anwendungsbeschränkt. Mit der in der WO 90/08196 beschriebenen Methode ist dagegen zwar eine Sensibilitätstestung mit verschiedenen Antibiotika in 15 - 24 h möglich. Jedoch ist das Verfahren zum allgemeinen Nachweis des Wachstums und zur Sensibilitätstestung von Mykobakterien mit dem in WO 90/08196 beschriebenen Verfahren nicht anwendbar, da der dort beschriebene Redoxindikator Resazurin mit dem Redoxstabilisator Kaliumhexacyanoferrat das sterile Medium allein bereits nach 48 - 72 h Inkubationszeit im Brutschrank von blau nach rot umschlagen läßt, also ein nicht vorhandenes Wachstum vortäuscht, wie durchgeführte Versuche zeigen. Grund hierfür sind die langen Inkubationszeiten bei Mykobakterien.
Zum Wachstumsnachweis betragen diese bei schnell wachsenden Mykobakterien etwa 1 Woche, während bei den langsam wachsenden Erregern der Tuberkulose, wie z.B. M. tuberculosis und M.bovis sowie bei dem bei AIDS-Patienten auftretenden Erreger M.avium, mindestens 8-10 Wochen zur Bebrütung erforderlich sind (DIN 58943, Teil 3).

Zur Empfindlichkeitstestung von Mykobakterien sind bei Bebrütung auf festen Nährmedien 4 Wochen erforderlich (DIN 58943 Teil 8), während bei flüssigen Nährmedien etwa 1 Woche benötigt wird.

Aufgabe vorliegender Erfindung ist es daher, ein Verfahren zum visuellen Nachweis des Wachstums von Mikroorganismen, der Empfindlichkeitsprüfung gegen Wirkstoffe sowie ihrer Identifizierung bereitzustellen, welches nicht auf bestimmte Antibiotika beschränkt ist und wobei das Testmedium so stabil ist, daß auch Organismen mit längeren Bebrütungszeiten wie z.B. Mykobakterien ohne falsch positive Ergebnisse nachgewiesen werden können.

Diese Aufgabe wird erfindungsgemäß dadurch gelöst, daß man dem Kultur- bzw. Testmedium eine Mischung aus den Indikatoren Methylenblau und Resazurin, vorzugsweise im Verhältnis von 1:4 bis 1:80, und in einer Menge, die für die Mikroorganismen ungiftig ist, wie z.B. 1-200 mg, insbesondere 1-20 mg Methylenblau bzw. 5-100 mg, insbesondere 7,5-50 mg Resazurin pro Liter Nährmedium, oder K₃ bzw. K₄Fe(CN)₆ in Verbindung mit FeII- bzw. FeIII-Salzen zusetzt.

Überraschenderweise wurde dabei festgestellt, daß mit der Indikatormischungischung aus Resazurin und Methylenblau einerseits der Wachstumsnachweis von Bakterien gezeigt als Farbumschlag von blau nach rot deutlich schneller erfolgt als mit Resazurin allein (z.B. für Enterobakteriaceae in 2 - 3 h, für Nonfermenter in 5 - 7 h, siehe nachfolgende Tabellen 1-5) und andererseits das sterile Medium an sich bis zu 4 Wochen und länger farbstabil blau bleibt.
Dies konnte im Hinblick auf die genannten Veröffentlichungen, insbesondere J. of Dairy Sciences, nicht erwartet werden. Denn dieser Offenbarung folgend konnte man erwarten, daß der Farbumschlag bei einer Mischung Methylenblau/Resazurin von blau nach rot wesentlich länger dauern würde, da zwar Resazurin schnell von blau nach rot umschlägt, aber das viel langsamere Methylenblau (Umschlag von blau nach farblos) nur eine violette Mischfarbe und einen nach Entfärbung des Methylenblaus entsprechend langsamen Umschlag nach rot ergeben würde. In Anwesenheit des Redoxstabilisators Kaliumhexacyanoferrat (der vorzeitiges Umschlagen durch das sterile Medium an sich verhindern soll und dies in dem Verfahren nach WO 90/08196 auch für 48 - 72 Stunden bewirkt) sollte dieser Farbumschlag, bewirkt durch bakterielles Wachstum, also noch deutlich langsamer erfolgen. Erfindungsgemäß erfolgt jedoch der Farbumschlag deutlich schneller.

Dies kann durch Zugabe eines Redoxstabilisators, wie z.B. Kaliumhexacyanoferrat [K₄Fe(CN)6] weiter verbessert werden, so daß das sterile Medium 2 - 3 Monate farbstabil blau bleibt (bei Bebrütung bei 35 - 37°C) und das Wachstum z.B. von Enterobakteriaceae dann immer noch in 3 - 4 h nachweisbar bleibt. Dies ermöglicht sowohl einen schnellen Nachweis des Wachstums und der Sensibilität von Mikroorganismen wie z.B. Enterobakteriaceae als auch den Nachweis des Wachstums und der Sensibilität gegen Antibiotika von langsam wachsenden Organismen wie Mykobakterien, der bisher nur auf festen Medien durch Wachstum von Kolonien oder in flüssigen Medien durch Einbau des radioaktiven Isotops C14 beschrieben ist.

Als Redoxstabilisatoren eignen sich insbesondere die Verbindungen K₃Fe(CN)₆, Tartrazingelb, Reactive Red 4, NH₄Fe(SO₄)₂, Malachitgrün sowie Kombinationen daraus mit dem oben beschriebenen K₄Fe(CN)₆. Die Stabilisatoren werden dabei in Mengen von 0,01-20%, insbesondere 0,1 bis 10% und ganz besonders von 1 bis 2,5% pro Liter Nährmedium zugesetzt.

Darüberhinaus wurde überraschenderweise gefunden, daß auch Mischungen von anorganischen Salzen des Fe(III), wie NH₄Fe(SO₄)₂ mit K₃Fe(CN)₆ bzw. des Fe(II) mit K₄Fe(CN)₆ in Kulturmedien als Redoxindikatoren zum Nachweis des Wachstums von Mikroorganismen, ihrer Identifizierung oder Sensibilitätstestung verwendet werden können, da in Abwesenheit von stoffwechselaktiven Organismen ein Farbumschlag von gelblich-braun über blaugrün nach blau erfolgt (es entsteht durch die von Mikroorganismen ausgelösten Redoxvorgänge Berliner Blau oder Turnbulls blue).

Die Verwendung dieser Kombination von Redoxindikatoren führt ebenso wie die Verwendung der Kombination von Methylenblau/Resazurin zu stabilen Kulturmedien, mit denen selbst nach längerer Zeit zuverlässig Mikroorganismen, insbesondere auch solche mit langen Bebrütungszeiten, wie Mykobakterien, nachgewiesen, identifiziert und ihre Empfindlichkeit gegen Antibotika getestet werden können. Dies war insofern nicht zu erwarten, da bisher Fe-haltige Indikatorsysteme nur zum Nachweis H₂S-produzierender Bakterien verwendet wurden. Es war daher überraschend, daß auch aerobe Organismen hiermit nachgewiesen werden können.

Ebenso wie bei der Kombination Methylenblau/Resazurin können dem Kulturmedium neben dem Fe-Indikationssystem die obengenannten Stabilisatoren zugesetzt werden. Die Mischung bleibt somit über Wochen stabil, ohne ihre Aktivität zu verlieren.

Besonders bevorzugte Fe-haltige Systeme sind Kombinationen aus NH₄Fe(SO₄)₂ und K₃Fe(CN)₆ sowie NH₄FeSO₄ und K₄Fe(CN)₆. Diese werden in Mengen von 1 bis 10%, vorzugsweise 0,1 bis 10% pro Liter Nährmedium, vorzugsweise im Verhältnis von 1:4 bis 1:10 (bezogen auf FeIII- oder FeII-Salz zu K₃ bzw. K₄Fe(CN)₆) verwendet.

Die erfindungsgemäße Methode läßt sich auf den Nachweis verschiedenster Mikroorganismen anwenden. Hierzu zählen insbesondere Hefen, Pilze, Nonfermenter, Kokken, Bazillen, Kokkobazillen sowie Enterobaktriaceae und bevorzugt die obengenannten Mykobakterien M. tuberculosis, M. avium, M. bovis.
Dabei kann ihre Empfindlichkeit gegenüber verschiedensten Wirkstoffen, wie z.B. Erythromycin, Tetracyclin, Penicillin, Oflaxacin, Oxacillin, Cefotaxim u.a.m. und insbesondere Tuberkulostatika wie Isoniazid, Dehydrostreptomycin und Ethambutol getestet werden.

Die Durchführung des Testverfahrens erfolgt beim Wachstumsnachweis der Mikroorganismen in Reagenzröhrchen mit etwa 3-4 ml Medium. Es können auch Mikrotiterplatten verwendet werden, wobei Standards mit 100 µl pro Vertiefung eingesetzt werden. Diese eignen sich auch zur Empfindlichkeitstestung und zur Identifizierungsreaktion der Mikroorganismen, wobei übliche Kulturmedien (z.B. Müller-Hinton-Medium) sowie Bebrütungsbedingungen und geeignete, dem Fachmann geläufige Variationen gewählt werden. Ferner werden übliche Puffer- und Nährmedien (z.B. Phosphat- Carbonatpuffer u.ä.) verwendet, wobei der pH-Wert im neutralen aber auch im sauren Bereich bzw. zwischen 6,4 und 8 liegen kann.

Die Erfindung wird anhand der nachfolgenden Beispiele näher erläutert.

### I. Beispiele 1-3: Empfindlichkeitsprüfung von normal wachsenden Bakterien gegen Antibiotika

1. 22,0 g Müller-Hinton Medium werden in 1 l 0,1 molarem Phosphat-Puffer pH 7,4 gelöst und autoklaviert. 0,005 g Resazurin und 422 mg K₄Fe(CN)₆, entspricht 0,001 molar, werden zugegeben und aufgelöst.
   Die entstandene Lösung wird als solche zur Sterilkontrolle benutzt, bzw. zur Auflösung verschiedener Antibiotika zur Sensitivitätstestung.
2. 22,0 g Müller-Hinton Medium werden in 1 l 0,1 molarem Phosphat-Puffer pH 7,2 gelöst und autoklaviert. 0,005 g Resazurin, 0,00125 g Methylenblau und 422 mg K₄Fe(CN)₆ werden zugegeben und gelöst.
   Die entstandene Lösung wird als solche zur Sterilkontrolle benutzt, bzw. zur Auflösung verschiedener nicht-β-Laktamantibiotika zur Sensitivitätstestung.
3. 22,0 g Müller-Hinton Medium werden in 1 l 0,1 molarem Phosphat-Puffer pH 6,8 gelöst und autoklaviert. 0,005 g Resazurin, 0,00125 g Methylenblau und 422 mg K₄Fe(CN)₆ werden zugegeben und gelöst.
   Die entstandene Lösung wird als solche zur Sterilkontrolle benutzt, bzw. zur Auflösung verschiedener β-Laktamantibiotika zu Sensitivitätstestung.

Die in den Beispielen 1-3 hergestellten Medien werden für die Bakterienstämme S.aureus ATCC 29213, E. coli ATCC 25922 und P.aeruginosa ATCC 27853 gegen Erythromycin, Tetracyclin, Ofloxacin, Cefotaxin und Penicillin G geprüft. Die Ergebnisse sind in nachfolgender Tabelle 1 zusammengefaßt, wobei Beispiel 1 den Vergleich gemäß WO 90/08196 und Beispiele 2 und 3 die Erfindung darstellen.

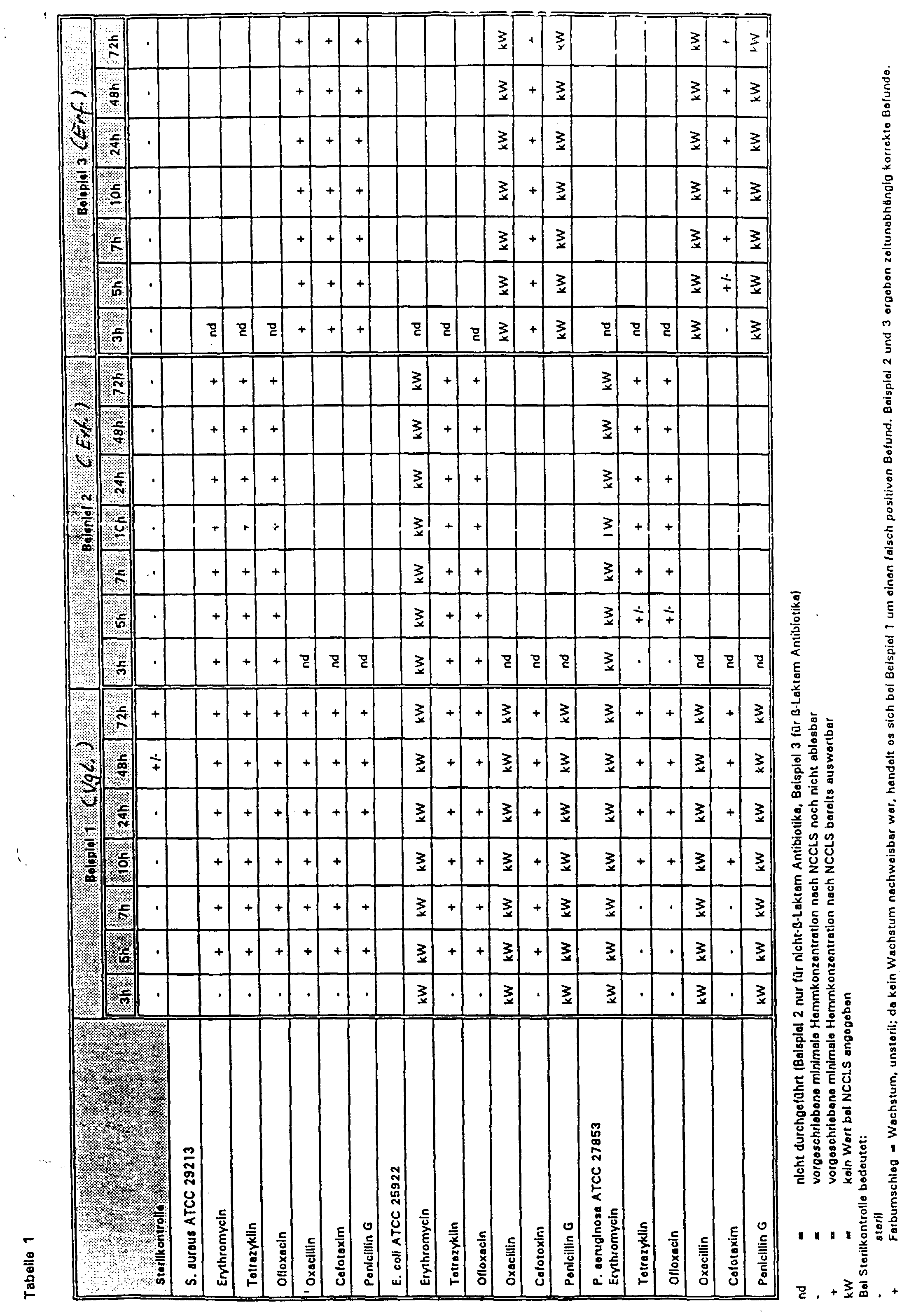

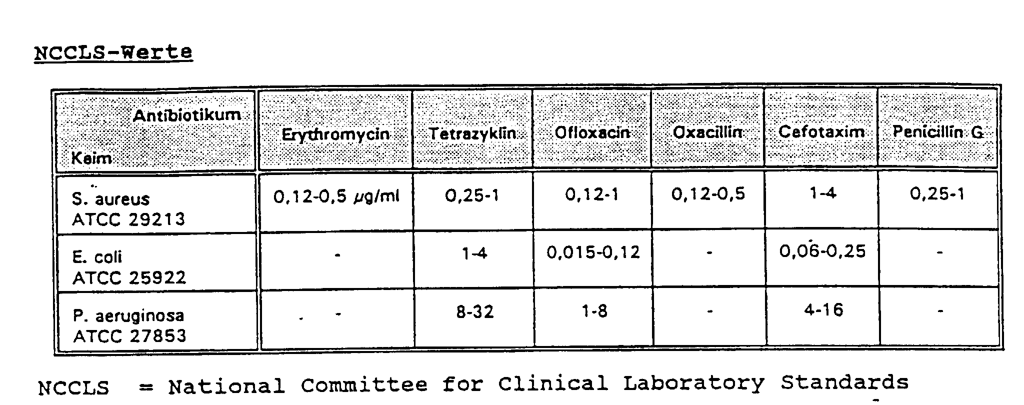

Tabelle 1 ist zu entnehmen, daß die Medien mit Methylenblau + Resazurin (Beispiel 2 und 3) die schnellere Erkennung des Antibiogramms ermöglichen. Ferner ist hieraus ersichtlich, daß die Sterilkontrolle länger spezifisch negativ (blau) bleibt und nicht unspezifisch (noch rot) umschlägt. Dies ermöglicht es, einen Ansatz erst wenige Tage später auszuwerten (z.B. Wochenendtests, ohne daß hierzu die Labors besetzt sein müssen). Solche Ergebnisse werden mit der bekannten Mischung, enthaltend nur Resazurin (Beispiel 1) nicht erhalten.

### II. Wachstumsnachweisverfahren für langsam wachsende Bakterien

### Beispiel 4: Herstellung eines Kulturmediums

11,0 g Müller-Hinton Medium werden in 1 l 0,1 molarem Phosphat Puffer pH 6,8 (für β-Laktamantibiotika) bzw. 1 l 0,1 molarem Phosphat Puffer pH 7,2 (für nicht β-Laktamantibiotika) gelöst und autoklaviert. 0,02 g Resazurin, 0,005g Methylenblau, 10 mg Reactive Red Nr. 4 und 422 mg K₄Fe(CN)₆ werden zugegeben und gelöst. Die entstandene Lösung wird als solche zur Sterilkontrolle benutzt bzw. zur Auflösung verschiedener Antibiotika zur Sensitivitätstestung. Mit dieser Zusammensetzung ergibt sich bei Evaluierung mit klinischen Isolaten und mehr verschiedenen Antibiotika eine bessere Korrelation zur Standardmethode nach NCCLS als mit der beschränkten Prüfkeimauswahl in den Beispielen 1 - 3.

### Beispiel 5a, b, c, d, e, f, g: Herstellung eines Flüssigmediums zum Nachweis von Mykobakterien

Eine Mischung aus folgenden Komponenten wird hergestellt:

| | |
|---|---|
| Middlebrook 7H9 broth Difco | 4,7 g |
| KH₂PO₄ | 3,5 g |
| Na₂KPO₄ | 2,5 g |
| L-Asparagin | 0,1 g |
| Kaliumaspartat | 3,0 g |
| Na-pyruvat | 0,5 g |
| Haemin (in NaOH vorgelöst) | 1,66 mg |
| Glucose | 0,5 g |
| Glycerin | 5 ml |
| Aqua dest. | 900 ml |
| | pH 6,40 |

Die Substanzen werden gelöst und 15 min. bei 121°C autoklaviert.Diesem Medium wird sterilfiltriert zugegeben:

| | |
|---|---|
| Kälberserum | 100 ml |
| Katalase-Lösung | 0,3 ml |
| Ribonukleinsäure (in Ethanol vorgelöst) | 25 mg |

Damit entsteht ein Basismedium mit Endvolumen 1 l pH 6,40
- Modifikation a:: + 20 mg Resazurin auf 1 l Medium
+ 5 mg Methylenblau " " " "
+ 1,69g K₄Fe(CN)₆ " " " "
- Modifikation b:: + 20 mg Resazurin auf 1 l Medium
+ 5 mg Methylenblau " " " "
+ 1,69g K₄Fe(CN)₆ " " " "
+ 1 mg Malachitgrün " " " "
- Modifikation c:: + 20 mg Resazurin auf 1 l Medium
+ 5 mg Methylenblau " " " "
+ 1,69g K₄Fe(CN)₆ " " " "
+ 20 mg Tartrazingelb" " " "
- Modifikation d:: + 20 mg Resazurin auf 1 l Medium
+ 5 mg Methylenblau " " " "
+ 1,69g K₄Fe(CN)₆ " " " "
+ 20 mg Reactive Red 4" " " "
- Modifikation e:: + 40 mg Resazurin auf 1 l Medium
+ 10 mg Methylenblau " " " "
+ 2,54g K₄Fe(CN)₆ " " " "
- Modifikation f:: + 40 mg Resazurin auf 1 l Medium
+ 10 mg Methylenblau " " " "
+ 2,54 g K₄Fe(CN)₆ " " " "
+ 1 mg Malachitgrün " " " "
+ 20 mg Tartrazingelb" " " "
- Modifikation g:: + 20 mg Resazurin auf 1 l Medium
+ 1,69g K₄Fe(CN)₆ auf 1 1 Medium

Die Medien 5a-g werden auf das Wachstum von Mykobakterien untersucht, wobei die Kombination 5 g (Resazurin allein) als Vergleich gemäß der WO 90/08196 dient. Die Ergebnisse sind in nachfolgender Tabelle 2 zusammengefaßt.

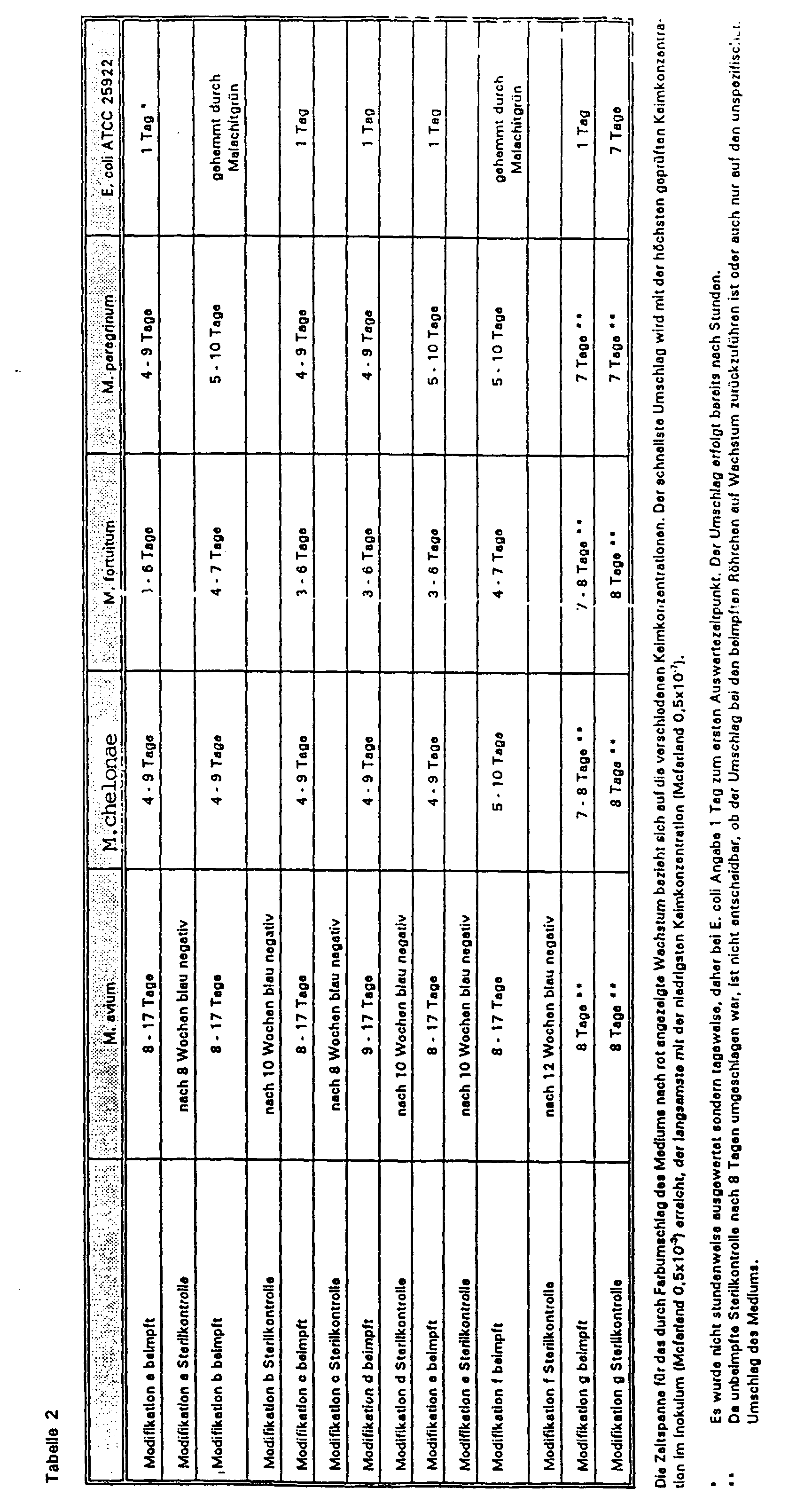

Den Ergebnissen in Tabelle 2 ist zu entnehmen, daß das Medium 5g (Vgl.), enthaltend nur Resazurin und K₄Fe(CN)₆ immer unspezifisch so früh nach rot umschlägt, daß nicht beurteilt werden kann, ob schnell oder langsam wachsende Mykobakterien in dem Medium gewachsen sind. Selbst schnell wachsende Mykobakterien wachsen nicht so schnell, daß der Farbumschlag eine Differenzierung von dem unspezifischen Effekt, der durch Bebrüten des sterilen Mediums eintritt, erlauben würde.

Bei allen anderen erfindungsgemäßen Modifikationen (5a - 5f) mit Methylenblau + Resazurin bleibt die unbeimpfte Sterilkontrolle nach 8 - 12 Wochen Bebrütung noch negativ (blau) und erlaubt damit eindeutig eine Differenzierung von dem durch Wachstum verursachten Farbumschlag nach rot. Dieser Wachstumsnachweis ist zudem bei dem langsam wachsenden Mykobakterium avium, welches auf festen Nährmedien ca. 3 - 6 Wochen benötigt, in 8 - 17 Tagen sehr schnell und empfindlich.

### III. Sensibilitätstest für langsam wachsende Mykobakterien

### Beispiel 6:

Hierzu wird das in Beispiel 5a beschriebene Kulturmedium, jedoch ohne Katalase-Lösung verwendet. Das Medium wird als solches zur Sterilkontrolle eingesetzt und zur Auflösung von den Antituberkulostatika Isoniazid in den Konzentrationen (0,03; 0,06; 0,125; 0,25; 0,5; 1,0; 2,0; 4,0 mg/L), Dehydrostreptomycin (0,5; 1,0; 2,0; 4.0; 8,0; 16,0; 32,0; 64,0 mg/L), und Ethambutol (0,125; 0,25; 0,5; 1,0; 2,0; 4,0; 8,0; 16,0 mg/L) eingesetzt. Die Lösungen werden in Mikrotiterplatten zu je 0,1 ml abgefüllt und eingefroren. Nach Auftauen werden sie mit einer Bakteriensuspension von 1mg/ ml sowie Verdünnungen dieser Suspension von 10-2, 10-3 und 10-4 beimpft. Die gleichen Bakteriensuspensionen von M. tuberculosis Stämmen werden zur Beimpfung konventioneller Löwenstein-Jensen Medien mit den gleichen Antituberkulostatika Konzentrationen verwendet. Es ergibt sich die gleiche Beurteilung der Stämme in resistent oder sensibel mit dem erfindungsgemäßen Medium, wie mit Löwenstein-Jensen Medium, jedoch ist das erfindungsgemäße Medium bereits nach 1 Woche ablesbar, während dies auf den konventionellen Medien 3 - 4 Wochen benötigt.

### IV. Identifizierungsreaktionen

### Beispiel 7:

Als Basismedium, um benötigte Aminosäuren, Vitamine und Mineralien für die Bakterien bereitzustellen, wird 100 millimolarer Phosphat-Puffer pH 7,2 mit 1 g/l Magnesiumsulfat und 1 g/l Ammoniumchlorid und 0,05 g/l Tryptone (Difco) verwendet. Anstelle von Tryptone kann auch Proteose-Peptone oder 0,04 g Tryptone (oder Proteosepepton) + 0,01 g Hefeextrakt verwendet werden. Als Puffer können weiter Carbonatpuffer oder Morpholinpropansulfonat oder ähnliche Puffer verwendet werden. Die zu katabolisierenden Substrate werden in einer Menge von ca. 20 mM/l eingesetzt. Als Wachstumsindikator enthält das Medium weiter 20 mg Resazurin, 5 mg Methylenblau und 1,69 g K₄Fe(CN)₆ pro Liter.

Die Ergebnisse sind in folgender Tabelle 3 dargestellt:

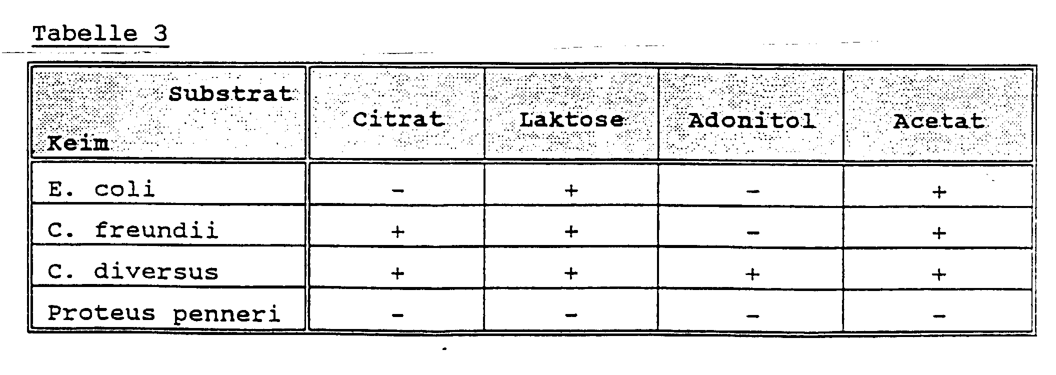

Wie hieraus ersichtlich ist, kann das erfindungsgemäße Indikatorgemisch auch zur Differenzierung der Keime bis zur Speziesebene (Identifizierung) eingesetzt werden.

### V. Fe-haltige bzw. Na₂WO₄-haltige Indikatormischungen

### Beispiel 8:

Zu dem in Beispiel 5 angegebenen Basismedium werden 1,2 g K₃Fe(CN)₆ und 4,8 g NH₄Fe(III)(SO₄)2 x 12H₂O zugesetzt.

Das sterile Medium behält bei Bebrüten seine gelbbräunliche Farbe. Das mit E. coli ATCC 25 922 beimpfte Medium schlägt durch den Stoffwechsel der wachsenden Bakterien nach blau um.

### Beispiel 9:

Zu dem in Beispiel 5 angegebenen Basismedium werden 2,94 g Na₂WO₄ x 2H₂O zugesetzt.

Das sterile Medium bleibt nach Bebrüten und Ansäuern mit 12%iger Schwefelsäure nach 48 Stunden gelblich.

Das mit E. coli ATCC 25 922 beimpfte Medium schlägt beim Ansäuern nach 48 Stunden durch den Stoffwechsel der wachsenden Bakterien nach blau um.

### VI. Stabilitätstests

In den folgenden Beispielen 10-13 wurden erfindungsgemäße Medien im Vergleich zur WO 90/08196-Methode ohne Redoxstabilisator untersucht.

### Beispiel 10: (Vgl. WO 90/08196, Müller-Hinton-Medium)

22,0 g Müller-Hinton Medium weden in 1 l 0,1 molarem Phosphatpuffer pH 7,4 gelöst und autoklaviert. 0,020 g Resazurin werden zugegeben und aufgelöst. Die entstandene Lösung wird als solche zur Sterilkontrolle benutzt bzw. zur Auflösung verschiedener Antibiotika zur Sensitivitätstestung.

### Beispiel 11: (Erf. Müller-Hinton-Medium)

22.0 g Müller-Hinton-Medium werden in 1 l 0,1 molarem Phosphatpuffer pH 7,4 gelöst und autoklaviert. 0,020 g Resazurin und 0,005 g Methylenblau werden erfindungsgemäß zugegeben und aufgelöst. Die entstandene Lösung wird als solche zur Sterilkontrolle benutzt bzw. zur Auflösung verschiedener Antibiotika zur Sensitivitätstestung.

Die Ergebnisse sind in nachfolgender Tabelle 4 zusammengefaßt.

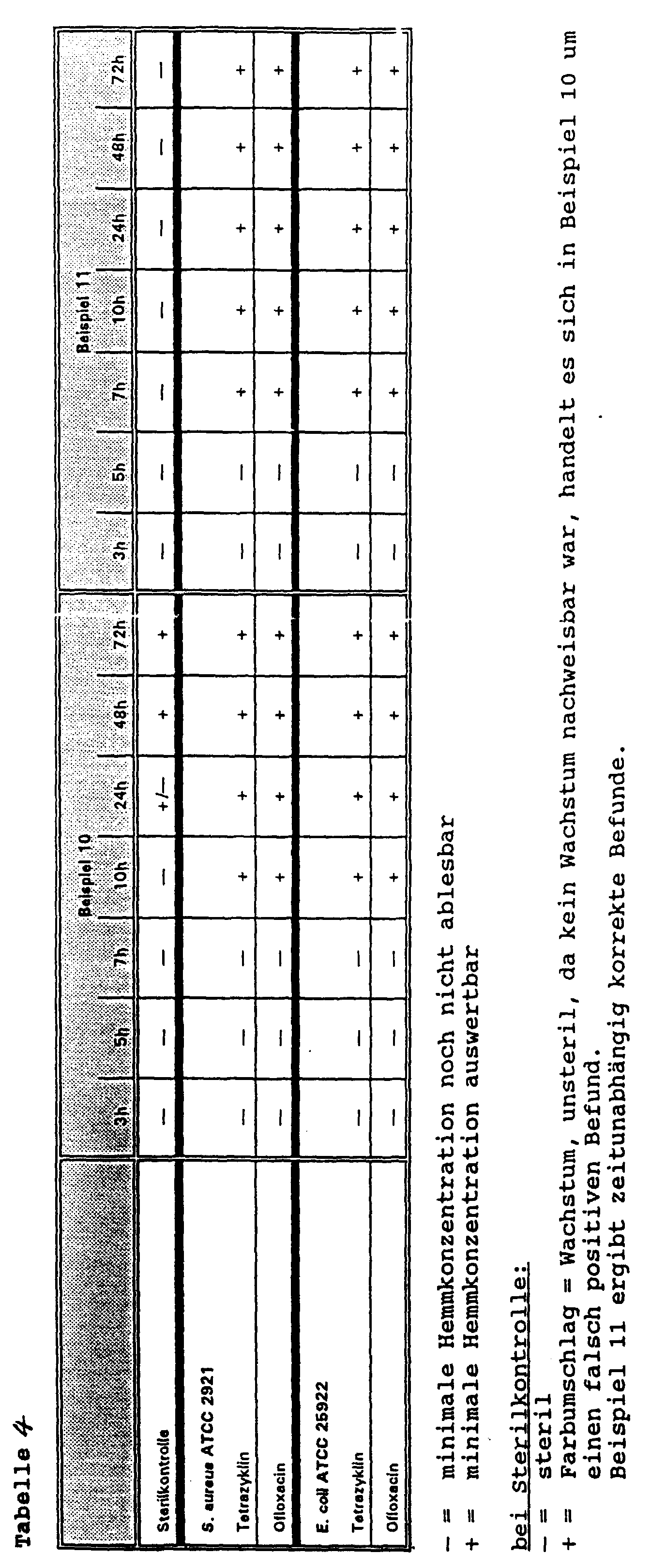

Aus Tabelle 4 ergibt sich folgendes.

In Beispiel 11 (Resazurin + Methylenblau gemäß vorliegender Erfindung) sieht man gegenüber Beispiel 10 (nur Resazurin; Vergl.), daß mit der erfindungsgemäßen Kombination ein stabiles Produkt erhalten wird, mit dem auch ohne Redoxstabilisator kein unspezifischer Umschlag erfolgt. Dieses Ergebnis ist mit der bekannten Mischung ohne Stabilisator nicht möglich.

### Beispiel 12: (Vgl.; Flüssigmedium für Mykobakterien)

Das Medium qemäß Beispiel 5a (Flüssigmedium für Mykobakterien) wird angesetzt. Auf 1 l Medium werden 20 mg Resazurin zugegeben.

### Beispiel 13: (Erf.; Flüssigmedium für Mykobakterien mit Resazurin)

Das Medium gemäß Beispiel 5a wird angesetzt. Auf 1 l Medium werden erfindungsgemäß 20 mg Resazurin und 5 mg Methylenblau zugegeben.

Die Ergebnisse dieses Vergleichsversuchs sind in Tablle 5 zusammengefaßt:

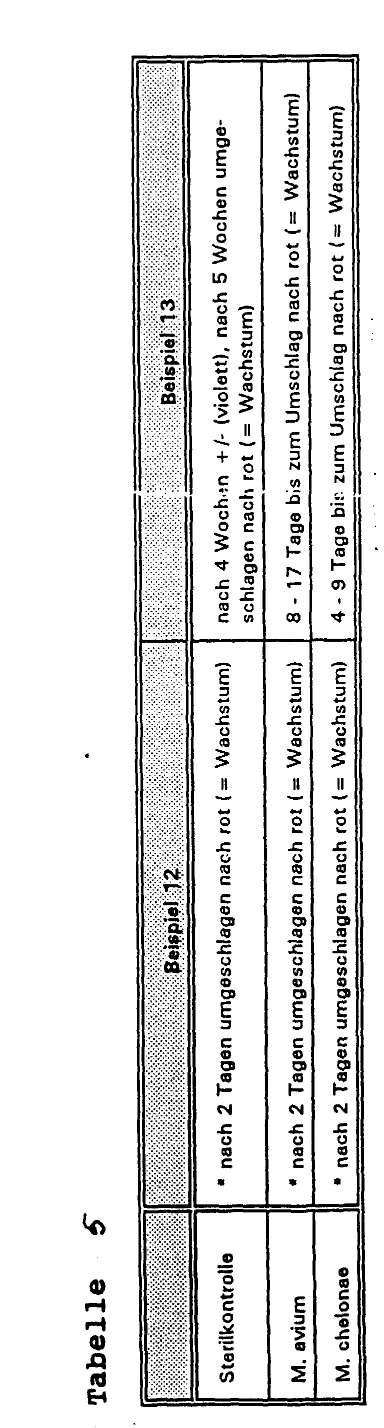

Die Zeitspanne für das durch Farbumschlag des Mediums nach rot angezeigte Wachstum bezieht sich auf die verschiedenen Keimkonzentrationen. Der schnellste Umschlag wird mit der höchsten geprüften Keimkonzentration im Ionokulum (McFarland 0,5x10⁻³) erreicht, der langsamste mit der niedrigsten Keimkonzentration (McFarland 0,5x10⁻⁷).

∗ Da die beimpfte Sterilkontrolle nach 2 Tagen umgeschlagen war, ist nicht entscheidbar, ob er Umschlag bei den unbeimpften Röhrchen auf Wachstum zurückzuführen ist oder auch nur auf den unspezifischen Umschlag des Mediums.

Tabelle 5 zeigt, daß mit der erfindungsgemäßen Kombination (Resazurin/Methylenblau, Beispiel 12) ein relativ stabiles Produkt erhalten wird, mit dem die Auswertung des Wachstums von schnell und langsam wachsenden Mykobakterien möglich ist. Das erfindungsgemäße Medium ist (Umschlag erst nach 5 Wochen = 35 Tagen) bereits 17-fach stabiler als die bekannte Mischung mit Resazurin (Beispiel 13), welche schon nach 2 Tagen einen Farbumschlag zeigt.

## Patentansprüche

1. Verfahren zum optischen Nachweis des Wachstums von Mikroorganismen, ihrer Identifizierung oder zur Durchführung der Empfindlichkeitsprüfung gegen Antibiotika, dadurch gekennzeichnet, daß man den Kulturmedien ein Gemisch aus den Redoxindikatoren Methylenblau und Resazurin zusetzt.

2. Verfahren zum optischen Nachweis des Wachstums von Mikroorganismen, ihrer Identifizierung oder zur Durchführung der Sensibilitätstestung, dadurch gekennzeichnet, daß man dem Kulturmedium Eisen(III)-Salze im Gemisch mit K₃Fe(CN)₆, Eisen(II)-Salze im Gemisch mit K₄Fe(CN)₆ zusetzt.

3. Verfahren nach Anspruch 1 oder 2, dadurch gekennzeichnet, daß man zusätzlich einen oder mehrere Redoxstabilisatoren, ausgewählt aus K₃Fe(CN)₆, Tartrazingelb, Reactive Red 4, Malachitgrün, NH₄Fe(SO₄)₂ sowie K₄Fe(CN)₆ oder Kombinationen daraus mit K₄Fe(CN)₆ verwendet.

4. Verfahren nach Anspruch 3, dadurch gekennzeichnet, daß man als Stabilisator K₄Fe(CN)₆ verwendet.

5. Verfahren nach einem der Ansprüche 1-4, dadurch gekennzeichnet, daß das Wachstum von Bakterien nachgewiesen wird.

6. Verfahren nach Anspruch 5, dadurch gekennzeichnet, daß man das Wachstum von Mykobakterien nachweist.

7. Verfahren nach Anspruch 1 oder 2, dadurch gekennzeichnet, daß man die Empfindlichkeit von Mykobakterien gegen Antituberkulostatika nachprüft.

8. Verfahren nach einem der Ansprüche 1 oder 3-7, dadurch gekennzeichnet, daß man Methylenblau und Resazurin im Verhältnis von 1:4 bis 1:80 verwendet.

9. Verfahren nach einem der Ansprüche 1 oder 3-8, dadurch gekennzeichnet, daß man Methylenblau in Mengen von 1-200 mg und Resazurin in Mengen von 5-100 mg pro Liter Nährmedium zusetzt.

10. Verfahren nach Anspruch 2, dadurch gekennzeichnet, daß man das Indikatorsystem in Mengen von 0,1-10% pro Liter Nährmedium zusetzt.

## Claims

1. Optical method of detecting the growth of microorganisms, identifying them or testing their sensitivity to antibiotics, characterized in that a mixture of the redox indicators methylene blue and resazurin is added to the culture media.

2. Optical method of detecting the growth of microorganisms, identifying them or testing their sensitivity, characterized in that a mixture of iron(III) salts with K₃Fe(CN)₆ or a mixture or iron(II) salts with K₄Fe(CN)₆ is added to the culture medium.

3. Method according to Claim 1 or 2, characterized in that one or more redox stabilizers, selected from K₃Fe(CN)₆, tartrazine yellow, reactive red 4, malachite green, NH₄Fe(SO₄)₂ and K₄Fe(CN)₆, or combinations thereof with K₄Fe(CN)₆, are additionally used.

4. Method according to Claim 3, characterized in that the stabilizer used is K₄Fe(CN)₆.

5. Method according to one of Claims 1 - 4, characterized in that the growth of bacteria is detected.

6. Method according to Claim 5, characterized in that the growth of mycobacteria is detected.

7. Method according to Claim 1 or 2, characterized in that the sensitivity of mycobacteria to antituberculostatics is tested.

8. Method according to one of Claims 1 or 3 - 7, characterized in that methylene blue and resazurin are used in a ratio of 1:4 to 1:80.

9. Method according to one of Claims 1 or 3 - 8, characterized in that methylene blue is added in amounts of 1 - 200 mg and resazurin in amounts of 5 - 100 mg per litre of nutrient medium.

10. Method according to Claim 2, characterized in that the indicator system is added in amounts of 0.1 - 10% per litre of nutrient medium.

## Revendications

1. Procédé pour la mise en évidence optique de la croissance de microorganismes, leur identification ou pour la mise en oeuvre d'un examen de sensibilité à des antibiotiques, caractérisé en ce que l'on ajoute aux milieux de culture un mélange des indicateurs redox bleu de méthylène et résazurine.

2. Procédé pour la mise en évidence optique de la croissance de microorganismes, leur identification ou pour la mise en oeuvre d'un test de sensibilité, caractérisé en ce que l'on ajoute au milieu de culture des sels de fer (III) en mélange avec K₃Fe(CN)₆, des sels de fer (II) en mélange avec K₄Fe(CN)₆.

3. Procédé selon la revendication 1 ou 2, caractérisé en ce que l'on utilise en outre un ou plusieurs stabilisateurs redox choisis parmi K₃Fe(CN)₆, le jaune de tartrazine, le Reactive Red 4, le vert malachite, NH₄Fe(SO₄)₂ ainsi que K₄fe(CN)₆ ou des combinaisons de ceux-ci avec K₄Fe(CN)₆.

4. Procédé selon la revendication 3, caractérisé en ce que l'on utilise K₄Fe(CN)₆ comme stabilisateur.

5. Procédé selon l'une des revendications 1 à 4, caractérisé en ce que la croissance de bactéries est mise en évidence.

6. Procédé selon la revendication 5, caractérisé en ce que l'on met en évidence la croissance de mycobactéries.

7. Procédé selon la revendication 1 ou 2, caractérisé en ce que l'on vérifie la sensibilité de mycobactéries à l'égard d'antituberculostatiques.

8. Procédé selon l'une des revendications 1 ou 3-7, caractérisé en ce que l'on utilise le bleu de méthylène et la résazurine dans le rapport de 1:4 à 1:80.

9. Procédé selon l'une des revendications 1 ou 3-8, caractérisé en ce que l'on ajoute le bleu de méthylène en des quantités de 1-200 mg et la résazurine en des quantités de 5-100 mg par litre de milieu nutritif.

10. Procédé selon la revendication 2, caractérisé en ce que l'on ajoute le système indicateur en des quantités de 0,1-10 % par litre de milieu nutritif.
